# EUROPÄISCHE PATENTSCHRIFT

(11) **EP 1 116 715 B1**
(45) Veröffentlichungstag und Bekanntmachung des Hinweises auf die Patenterteilung: **06.10.2004**
(21) Anmeldenummer: 00100457.1
(22) Anmeldetag: 10.01.2000
(51) Int. Cl.: C07D 207/38, A61K 31/4015, A61P 31/00, C12P 17/10, C12R 1/225, A23L 1/00

(54) **Tetramsäure-Derivate zur Verwendung in Medizin und Lebensmitteltechnologie**
Tetramic acid derivatives useful in medicines and foodstuffs
Dérivés d'acide tetramique et l'utilisation dans des produits médicinales et alimentaires

(43) Veröffentlichungstag der Anmeldung: 18.07.2001
(73) Patentinhaber: Jung, Günther, Prof. Dr., 72076 Tübingen (DE); Stracke, Friedrich, Dr., 72074 Tübingen (DE); Wiesmüller, Karl, Dr., 72070 Tübingen (DE)
(72) Erfinder: Jung, Günther, Prof. Dr, 72076 Tübingen (DE); Hammes, Walter, Prof. Dr., c/o Univ. Hohenheim, Garbenstrasse 25, 70593 Hohenheim (DE); Gänzle, Michael, Dr, c/o TU München, Weihenstephaner Steig 16, 85350 Freising (DE); Marquardt, Udo, Dipl.- Chem., 72076 Tübingen (DE); Höltzel, Alexandra, Dr., 72076 Tübingen (DE)
(74) Vertreter: Patentanwälte Ruff, Wilhelm, Beier, Dauster & Partner

(56) Entgegenhaltungen:
- DE-A- 2 352 448
- US-A- 5 527 820

## Beschreibung

Die Erfindung betrifft neue Verbindungen und Verfahren zur Herstellung dieser Verbindungen, sowie ihre Verwendung.

Die Ausnutzung der Milchsäuregärung durch Milchsäurebakterien ist ein traditionelles Verfahren zur Haltbarmachung von Lebensmitteln. Durch Milchsäuregärung haltbar gemachte Lebensmittel, sogenannte fermentierte Lebensmittel (Fermentation = Gärung), finden sich überall auf der Welt. Beispiele für solche Lebensmittel sind Käse, Joghurt, andere Milchprodukte, Brot und Sojasoße.

Die ökologischen Bedingungen während der Lebensmittelfermentierung gewähren Milchsäurebakterien einen selektiven Vorteil, so daß die Milchsäurebakterien die dominierende Mikroflora in fermentierten Lebensmitteln ausmachen.

Die Vertreter der Milchsäurebakterien sind morphologisch relativ uneinheitlich, physiologisch sind sie jedoch gut zu charakterisieren. Das charakteristische Stoffwechselprodukt dieser Bakterien ist die Milchsäure (Lactat). Die Milchsäure wird über die sogenannte homofermentative oder heterofermentative Milchsäuregärung im Ergebnis aus Glukose katabolisiert. Durch dieses Stoffwechselprodukt erniedrigen die Milchsäurebakterien den pH-Wert ihres Substrates innerhalb kurzer Zeit auf Werte unterhalb von 5 und unterdrücken dadurch das Wachstum anderer anaerober Bakterien, die bei diesem Säuregrad nicht zu wachsen vermögen. Welche Milchsäurebakterien sich in diesen Anreicherungskulturen durchsetzen, ist von den jeweiligen Bedingungen abhängig. Der auf der Ansäuerung beruhenden sterilisierenden und konservierenden Wirkung verdanken die Milchsäurebakterien ihre Verwendung in der Landwirtschaft, im Haushalt, in der Molkerei und in der milchverarbeitenden Industrie. Ihre Verwendung gewährleistet die hygienische Sicherheit und die Lagerungsstabilität der verschiedenen Lebensmittel und trägt daneben zum charakteristischen Geschmack, Aroma und Struktur der Produkte bei.

Weiterhin gehören Milchsäurebakterien zu den Lebensmittelkomponenten mit bewiesener physiologischer Wirkung. Sie sind Bestandteil einer sogenannten funktionellen Nahrung (probiotische Lebensmittel), die als jede Art veränderter Nahrung oder Nahrungsbestandteil definiert ist, welche einen Gesundheitsvorteil zusätzlich zu den darin enthaltenen Nährstoffen bewirkt. Funktionelle Nahrung verbessert oder bewirkt somit Körperfunktionen über die normalen Nährstoffe hinaus. Seit langem sind für die Joghurtherstellung und für ähnliche Lebensmittel sowie für Sauerteig spezielle Milchsäurestämme in der Anwendung. Die Milchprodukte besitzen mit 55 % den größten Anteil an der funktionellen Nahrung, welche in immer stärkerem Maße vor allem in den Industrieländern eingesetzt wird.

Die Wirkung von Milchsäurebakterien beruht auf verschiedenen spezifischen und unspezifischen inhibitorischen Prinzipien. Hierzu zählt die Akkumulierung von organischen Säuren und die Ansäuerung des Substrates durch die Bakterien. Darüber hinaus werden von verschiedenen Milchsäurebakterien sogenannte Bakteriocine produziert. Dies sind Protein- oder Peptid-Komponenten, die antagonistisch, d.h. hemmend, auf andere Bakterienarten wirken, die zum Teil den jeweiligen Milchsäurebakterien sehr nahe verwandt sein können. Aufgrund dieser antagonistischen Aktivitäten gegen andere Mikroorganismen, z.B. auch gegen verschiedene Pathogene, können Milchsäurebakterien auch zur Konservierung von Lebensmitteln eingesetzt werden, die normalerweise nicht für eine herkömmliche Fermentation geeignet sind.

Neben der Verwendung in der Lebensmitteltechnologie sind die von Milchsäurebakterien produzierten antagonistischen Aktivitäten, insbesondere die Bakteriocine auch für medizinische Anwendungen als Antibiotikum von großem Interesse. Allerdings sind Bakteriocine in der Regel durch ein sehr enges Wirkungsspektrum gekennzeichnet, so daß eine breitere Anwendung in der Regel nicht möglich ist.

Es wurden bereits antimikrobielle Aktivitäten von Milchsäurebakterien beobachtet, die nicht auf peptidische Bakteriocine oder auf organische Säuren zurückzuführen sind. Derartige Sekundärmetaboliten konnten bisher jedoch weder auf molekularer Ebene charakterisiert noch gereinigt werden.

DE-A-2 352 448 beschreibt das Antibiotikum Magnesidin und ihre Anwendung in pharmaseutischen Zubereitungen und in der hebensmittelindustrie.

Die Erfindung stellt sich daher die Aufgabe, eine nichtpeptidische antimikrobielle Aktivität aus Milchsäurebakterien zu identifizieren und zu charakterisieren, und diese neue Komponente für eine Vielzahl von Anwendungen und Einsatzgebieten bereitzustellen. Als Einsatzgebiete sind vor allem die Lebensmitteltechnologie und die Verwendung in medizinischen Anwendungen als Antibiotikum von Interesse. Die Aufgabe wird gelöst durch Verbindungen, wie sie im Anspruch 1 dargestellt sind. Bevorzugte Ausführungsformen dieser Verbindungen sind in den Ansprüchen 2-10 beansprucht. Die Ansprüche 11-20 betreffen die Verwendung dieser Verbindungen in der Medizin und in der Lebensmitteltechnologie sowie entsprechende pharmazeutische Zusammensetzungen und probiotische Lebensmittel. Durch die Verfahren zur Isolierung bzw. zur Herstellung dieser Verbindungen, welche in den Ansprüchen 21 und 22 beansprucht sind, werden die Verbindungen für jegliche Anwendungen bereitgestellt. Der Wortlaut sämtlicher Ansprüche wird hiermit durch Bezugnahme zum Inhalt der Beschreibung gemacht.

Bei der neuen antimikrobiellen Aktivität aus Milchsäurebakterien handelt es sich um eine Verbindung gemäß der Formel I.

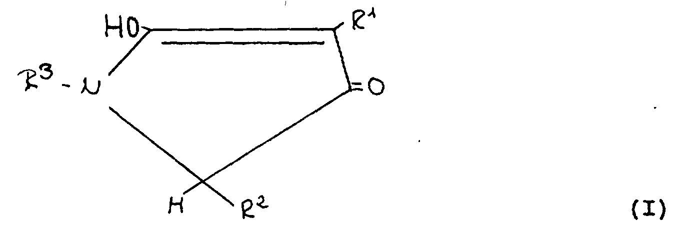

Diese Verbindung ist ein strukturell neuartiger Heterozyklus, welcher sich den Tetramsäurederivaten zuordnen läßt. An den Ringpositionen 3, 5 und 1, dem Stickstoff, befinden sich drei Reste (R¹, R² und R³). Bei R¹ handelt es sich um einen Acylrest, welcher vorzugsweise ein Acylrest einer organischen Carbonsäure ist. R² ist ein Alkylrest, vorzugsweise ein Alkylrest mit einem bis fünf C-Atomen. R³ ist ein Acylrest einer organischen Carbonsäure.

Diese Verbindung wurde als niedermolekularer, im wesentlichen antibiotisch wirksamer Naturstoff aus Milchsäurebakterien isoliert und spektroskopisch aufgeklärt. In Anlehnung an einen produzierenden Stamm (Lactobacillus reuteri) wurde die erfindungsgemäße Verbindung als Reutericyclin benannt. Der Produzentenstamm wurde 1990 aus Sauerteig isoliert und befindet sich in der Stammsammlung der Universitäten Hohenheim und München wobei der Stamm aus kommerziell erhältlichen Starterkulturen der Firma Böcker (Minden, Deutschland) gewonnen wurde. Die Starterkulturen werden als Lebendkulturen oder fermentiert und trocken vertrieben.

An Position 5 des Heterozyklus befindet sich ein Chiralitätszentrum. In einer bevorzugten Ausführungsform der Erfindung stellt die Verbindung ein Gemisch aus den beiden möglichen Enantiomeren dar (Racemat). In einer besonders bevorzugten Ausführungsform handelt es sich bei der Verbindung jedoch im wesentlichen um eine enantiomerenreine Form (insbesondere die R-Form). Die enantiomerenreine Verbindung zeigt im allgemeinen eine höhere antimikrobielle Aktivität, kann jedoch andererseits in der Herstellung aufwendiger sein als das Racemat.

In einer bevorzugten Ausführungsform der Erfindung ist der Rest R¹ ein Acetylrest. Der Rest R² ist vorzugsweise ein Butylrest, insbesondere ein Isobutylrest. Für die antimikrobielle Aktivität der erfindungsgemässen Verbindung sind diese Reste R¹ und R² jedoch nicht zwingend kritisch, so daß auch andere Reste in diesen Positionen bevorzugt sein können.

Von besonderer Bedeutung für die antimikrobielle Aktivität der Verbindung ist in der Regel der Rest R³. Hierbei handelt es sich um einen Acylrest einer organischen Carbonsäure, welcher vorzugsweise der Rest einer längerkettigen Carbonsäure ist. In einer besonders bevorzugten Ausführungsform weist dieser Rest acht bis zwölf C-Atome auf. Besonders bevorzugt ist ein Rest mit zehn C-Atomen.

In einer weiteren bevorzugten Ausführungsform der Erfindung handelt es sich bei dem Rest R³ um den Rest einer ungesättigten Carbonsäure, vorzugsweise einer einfach ungesättigten Carbonsäure. Besonders bevorzugt ist eine α,β -ungesättigte Carbonsäure.

Dieser Rest R³ ist vorzugsweise Decenoyl, vorzugsweise 2-trans-Decenoyl.

Die chemische Bezeichnung einer bevorzugten erfindungsgemässen Verbindung lautet 1-(2-trans-Decenoyl)-3-Acetyl-5-Isobutyl-Δ₂-Pyrrolin-4-on. Dies ist das razemische Gemisch der erfindungsgemässen Verbindung. Wie bereits erwähnt, kann die enantiomerenreine Form bevorzugt sein. Hierbei ist besonders die Verbindung 5R-1-(2-trans-Decenoyl)-3-Acetyl-5-Isobutyl-Δ ₂-Pyrrolin-4-on vorteilhaft.

Neben den dargestellten erfindungsgemässen Verbindungen sind selbstverständlich auch die Salze dieser Verbindungen sowie die verschiedenen Enolprodukte bzw. Tautomerieformen dieser Verbindungen mit von der Erfindung umfaßt.

Die chemische Struktur des Reutericyclin genannten Naturstoffes, welcher aus dem Stamm Lactobacillus reuteri LTH2584 zunächst isoliert wurde, wurde durch verschiedene NMR-spektroskopische Experimente und GC-MS-Analysen von Methanolyse-Produkten aufgeklärt. Näheres hierzu ist aus den Ausführungsbeispielen zu entnehmen. Die Struktur des Naturstoffes sowie der anderen wirksamen Verbindungen wurde durch Synthesen belegt und weiter charakterisiert, wie ebenfalls aus den Beispielen ersichtlich wird.

Bei dem aus Lactobacillus reuteri LTH2584 isolierten Naturstoff handelt es sich um eine 3-Acyltetramsäure mit neuartiger Substitution des Ringstickstoffes durch eine α ,β-ungesättigte Fettsäure (Acylrest einer organischen Carbonsäure). Der im Naturstoff gefundene 2-Decenoyl-Substituent an dieser Position N-1 ist offensichtlich für die veränderten chemischen und biologischen Eigenschaften von Reutericyclin gegenüber anderen natürlich auftretenden oder bereits bekannten (US-A-3299095) 3-Acyltetramsäuren verantwortlich. Reutericyclin ist amphiphil und liegt in Lösung bevorzugt als endozyklische Δ₂-Pyrrolin-4-on-Form vor.

Reutericyclin wurde als antimikrobielle Aktivität aus Milchsäurebakterien isoliert und identifiziert. Aufgrund dieser Eigenschaft der Verbindung sowie der verschiedenen Analoga, die oben dargestellt sind, werden diese Verbindungen vorzugsweise als Antibiotikum, also Substanzen, die hemmend auf andere Organismen, vorzugsweise Mikroorganismen, wirken, verwendet. Besonders vorteilhaft werden die erfindungsgemässen Verbindungen gegen das Wachstum von Gram-positiven Bakterien eingesetzt. Beispiele für die Wirksamkeit von den erfindungsgemässen Verbindungen ergeben sich aus den Ausführungsbeispielen.

Besonders bevorzugt ist der Einsatz der erfindungsgemässen Verbindungen gegen das Wachstum von Vancomycin-resistenten Keimen. Diese in letzter Zeit vermehrt auftretenden Keime sind sehr schwer durch herkömmliche Antibiotika zu behandeln, so daß der Einsatz der hier beschriebenen neuen Verbindungen von ganz besonderer Bedeutung ist.

In einer weiteren bevorzugten Ausführungsform der Erfindung werden die erfindungsgemässen Verbindungen im Bereich der Lebensmitteltechnologie verwendet. Die antimikrobiellen Eigenschaften der Verbindungen sorgen hierbei dafür, dass verunreinigende Mikroorganismen, insbesondere Pathogene, abgetötet bzw. in ihrem Wachstum gehemmt werden. Hierdurch wird die Haltbarkeit der Lebensmittel erhöht und darüber hinaus schädliche Wirkungen beim Verzehr der Lebensmittel vermieden.

Besonders bevorzugt ist die Verwendung der erfindungsgemässen Verbindungen zur Herstellung von probiotischen Lebensmitteln. Im allgemeinen üben die sogenannten probiotischen Lebensmittel einen über den eigentlichen Nährstoffgehalt der Lebensmittel hinausgehenden positiven Effekt auf die menschliche Gesundheit aus. In dieser Hinsicht sind die erfindungsgemässen Verbindungen besonders vorteilhaft einsetzbar, da sie zum einen für die Lebensmittelkonservierung und zum anderen zur gezielten Beeinflussung beispielsweise der menschlichen Intestinalflora geeignet sind.

In einer Ausführungsform der Erfindung werden die erfindungsgemässen Verbindungen als Zusatz (Additive) in den Lebensmitteln verwendet. Andererseits kann es auch vorteilhaft sein, die Verbindungen beispielsweise für die Konservierung der Lebensmittel einzusetzen, um sie anschließend wieder zu entfernen, bevor die Lebensmittel verzehrt werden.

Die erfindungsgemässen Verbindungen sind in einem relativ breiten Konzentrationsspektrum einsetzbar. Eine Toxizität der Verbindungen konnte bisher nicht beobachtet werden, so daß auch höhere Konzentrationen der erfindungsgemässen Verbindungen zumindest derzeit als unschädlich zu betrachten sind. Um die erfindungsgemässen Wirkungen zu erzielen, wird es jedoch in der Regel sinnvoll sein, Konzentrationen von etwa 0,01 bis etwa 1 mg/l einzusetzen. Besonders bevorzugt sind Konzentrationsbereiche von etwa 0,05 bis etwa 0,15 mg/l. Dies gilt insbesondere für den Einsatz der Verbindungen als Antibiotikum. Bei den zu wählenden Konzentrationen werden jedoch im allgemeinen die gewünschten Effekte zu berücksichtigen sein. So zeigen verschiedene Mikroorganismen unterschiedlich starke Empfindlichkeiten gegenüber den erfindungsgemässen Verbindungen, so daß unterschiedliche Konzentrationen für die Abtötung dieser Mikroorganismen vorteilhaft sind. Weiterhin können wirksame Konzentrationen auch von anderen Faktoren abhängig sein, beispielsweise von weiteren Komponenten innerhalb der eingesetzten Gemische oder von anderen Faktoren wie Temperatur oder pH-Wert.

In einer bevorzugten Ausführungsform der Erfindung werden die erfindungsgemässen Verbindungen zur Herstellung einer pharmazeutischen Zusammensetzung eingesetzt. Diese erfindungsgemässen pharmazeutischen Zusammensetzungen dienen in der Regel als Antibiotikum, mit dessen Hilfe bestimmte Erreger im Menschen oder Tier abgetötet werden oder ganz generell in ihrem Wachstum oder ihrer Ausbreitung beeinflußt werden sollen. Solche Zusammensetzungen können jedoch auch für andere Zwecke eingesetzt werden, beispielsweise für Zellkulturen oder ähnliches.

Die Erfindung umfaßt ferner die Verwendung der erfindungsgemässen Verbindungen zur Herstellung von Lebensmitteln, insbesondere zur Herstellung von probiotischen Lebensmitteln. Zusätzlich werden Lebensmittel, insbesondere probiotische Lebensmittel, die mindestens eine verbindungsgemässe Verbindung aufweisen, von der Erfindung umfaßt.

Um ausreichende Mengen des Naturstoffs Reutericyclin, insbesondere 5 R-1-(2-trans-Decenoyly-3-Acetyl-5-Isobutyl-Δ₂-Pyrrolin-4-on, für die verschiedenen Verwendungen bereitstellen zu können, umfaßt die Erfindung ein Verfahren zur Isolierung dieses Stoffes aus Lactobacillus reuteri, vorzugsweise aus Lactobacillus reuteri LTH2584.

Darüber hinaus umfaßt die Erfindung ein Verfahren zur Herstellung, bzw. zur Synthese des Naturstoffs und der verschiedenen Analoga, die oben beschrieben sind. Das erfindungsgemässe Herstellungsverfahren konnte zusätzlich dazu genutzt werden, um die Richtigkeit der Strukturaufklärung des Naturstoffes zu bestätigen. Das Herstellungsverfahren umfaßt eine 7-stufige Synthese, die im wesentlichen aus drei wichtigen Syntheseschritten besteht.

Dies ist zunächst eine C-Acylierung von Meldrumsäure mit vorzugsweise einer Aminosäure, die einen entsprechenden Acylrest, insbesondere Fettsäurerest aufweist. Besonders bevorzugt ist hierbei eine (2-trans-Decenoyl)-Aminosäure, insbesondere (2-trans-Decenoyl)-Leucin. Durch die Auswahl des Fettsäurerestes bzw. der jeweiligen Aminosäure werden die Reste R³ bzw. R² bestimmt. Somit kann hierdurch die Struktur des letztendlichen Produktes variiert werden. An die C-Acylierung der Meldrumsäure schließt sich eine thermische Cyclisierung und anschließend eine Acylierung in C3-Position an. Die C3-Acylierung wird vorzugsweise als C3-Acetylierung durchgeführt. Der Acylrest, der an dieser Position eingeführt wird, kann jedoch variiert werden, um so das gewünschte Endprodukt zu erhalten.

Näheres zum Herstellungsverfahren der erfindungsgemässen Verbindungen sowie weitere Merkmale der Erfindung ergeben sich aus der folgenden Beschreibung von Ausführungsbeispielen in Verbindung mit den Unteransprüchen, wobei die individuellen Merkmale jeweils für sich und in Kombination miteinander beansprucht sind.

In den Abbildungen zeigt:
- Figur 1: Schematische Darstellung der Reutericyclin-Tautomere. Durch NMR-spektroskopische Experimente erhaltene Strukturfragmente sind fett gezeichnet;

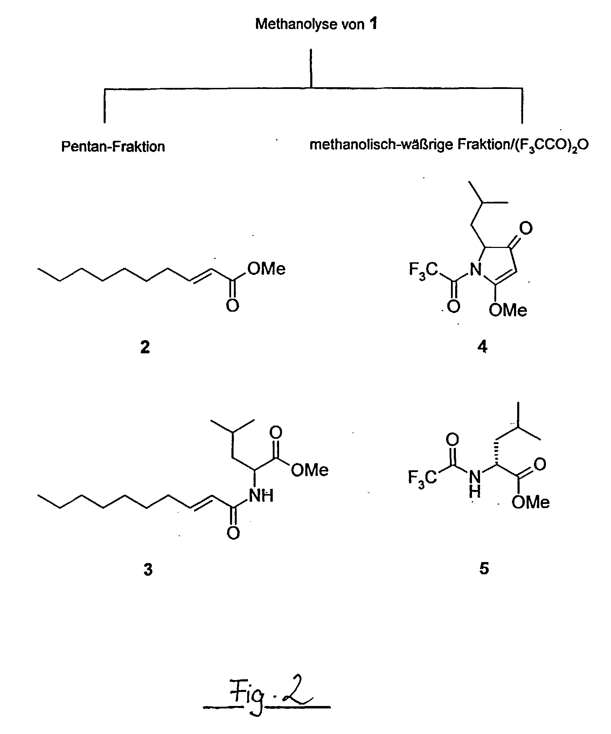

- Figur 2: Durch GC-MS-Analyse identifizierte Methanolyseprodukte von Reutericyclin, detektiert bei m/z 184 **(2),** 297 **(3),** 265 **(4)** und 241 **(5);**
- Figur 3: Synthese des Reutericyclins: a) CH₂(COOH)₂, -CO₂ b) SOCl₂ c) Leu-OtBu d) H₂O, H⁺ e) Meldrumsäure, DCC, DMAP f)Δ, - Aceton, -CO₂ g) Ac-Cl, TiCl₄;
- Figur 4: Formelbild von Reutericyclin mit Numerierung der Kohlenstoffatome;
- Figur 5: 13C-NMR-Übersichtsspektrum von Reutericyclin (Lösungsmittel DMSO-d₆, 16 mg/ml);
- Figur 6: ¹³C-NMR-Spektrum der Methyl- und Methylensignale (Ausschnitt von Figur 5). Die Signale von C-7 (überlagert durch DMSO-d₆-Signal) und C-6 (aufgrund von Tautomerieeffekten) sind im 1D-Spektrum nicht sichtbar.

### Beispiele

### Beispiel 1: Isolierung von Reutericyclin aus Lactobacillus reuteri LTH2584

Der Stamm Lactobacillus reuteri LTH2584 wurde als einer der dominierenden Fermentationskeime aus einem Sauerteig isoliert, der zur Produktion eines kommerziell erhältlichen Backmittels hergestellt wird.

Reutericyclin wurde als gelbbraunes Öl aus dem Kulturüberstand und aus dem Zellextrakt von L. reuteri LTH2584 in einer Ausbeute von etwa 1 mg/l isoliert.

Die Reinigung umfaßte die Extraktion von Zellen mit Phosphatpuffer (50 mM, pH 6,5)/Isopropanol 70/30, gefolgt von Gelpermeationschromatographie an Superdex S30 (Pharmacia, Triethylaminpuffer (50 mM, pH 7,2)/Isopropanol 75/25) und Mitteldruckchromatographie an einer Umkehrphase (Pharmacia, 15 µm, Eluent A: H₂O/O,1 % TFA, Eluent B: Isopropanol/0,1 % TFA, Gradient: 25 % nach 50 % B in 20 min). Die Feinreinigung erfolgte durch HPLC (Advanced Separation Technologies, C₁₈, 5 µm, Eluent A: Acetonitril/0,1 % TFA, Eluent B: H₂O/0,1 % TFA, A/B: 85/15).

### Beispiel 2: Massenspektroskopische Analyse von Reutericyclin

Die Summenformel von Reutericyclin **1** wurde durch hochauflösende ESI-FT-ICR-Massenspektrometrie (ICR=Ionenzyklotronresonanz) im Kationenmodus anhand des Quasi-Molekülions [M+H]⁺ bei m/z 350.23210 (berechnet 350.23257) zu C₂₀H₃₁N0₄ bestimmt. Das UV/Vis-Spektrum (Acetonitril) wies Absorptionsmaxima bei 238 nm und 286 nm auf. Eine intensive Bande bei 1726 cm⁻¹ sowie weitere starke Absorptionen zwischen 1657 cm⁻¹ und 1617 cm⁻¹ im IR-Spektrum (Film) belegten die Anwesenheit mehrerer Carbonylgruppen im Molekül. Die Strukturaufklärung von **1** erfolgte auf der Basis von NMR-spektroskopischen Experimenten sowie der GC-MS Analyse von Methanolyseprodukten.

Im HSQC-Spektrum wurden Signale für vier Methyl-, sieben Methylen-, zwei aliphatische und zwei olefinische Methingruppen identifiziert. Im ¹³C-NMR-Spektrum traten zusätzlich Signale für fünf quartäre Kohlenstoffatome auf, von denen zwei als zu Ketogruppen und zwei als zu Säure-, Ester- oder Amidfunktionen gehörig klassifiziert wurden (Tabelle 1).

Über TOCSY-Experimente (unter Verwendung kurzer und langer Mischzeiten) wurden die Resonanzen zweier Spinsysteme, die jeweils H-5 bis H-9 und H-13 bis H-21 umfassen, zugeordnet. Die E-Konfiguration der Doppelbindung zwischen C-13 und C-14 wurde durch die vicinale Kopplungskonstante der Protonen H-13 und H-14 von ³J_{HH}-15,4 Hz angezeigt.

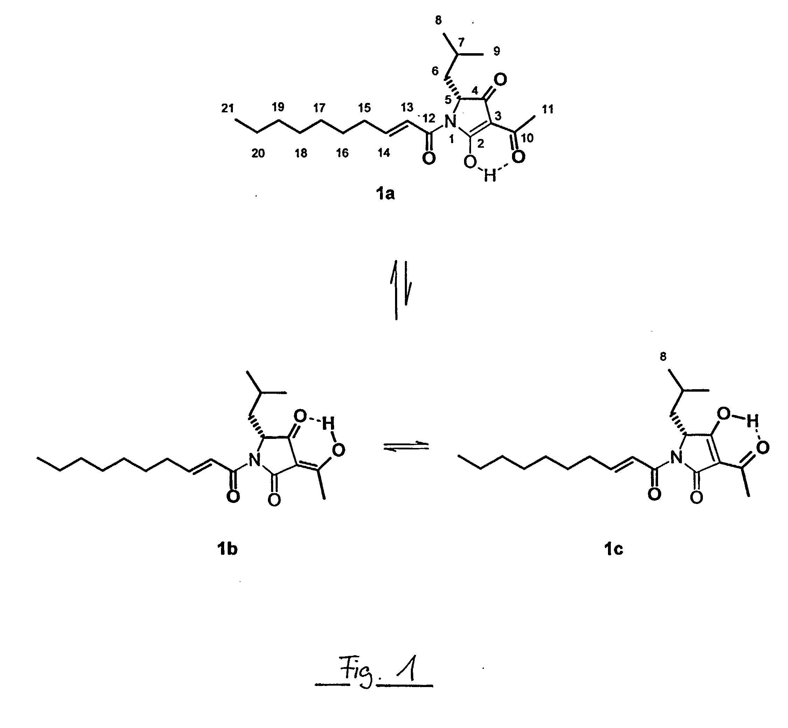

Die im HMBC-Spektrum beobachteten Konnektivitäten (Tabelle 1) ermöglichten die Erweiterung der beiden Spinsysteme um die quartären Kohlenstoffatome C-4 und C-12 zu den in Abbildung 1 dargestellten Strukturfragmenten. Ein drittes Fragment ergab sich aus den HMBC-Korrelationen der Methylprotonen H-11 zu den quartären Kohlenstoffatomen C-3 und C-10, wobei die ¹H-chemische Verschiebung der Methylprotonen H-11 von delta(¹H)=2,51 für die direkte Nachbarschaft von Methylgruppe und Ketofunktion sprach. HMBC-Korrelationen zwischen den etablierten Fragmenten oder zu einem weiteren Carbonylkohlenstoffatom (C-2) waren nicht vorhanden.

Das saure Methanolysat (CH₃OH/1,5 N HCl, 110°C, 15 min) von **1** wurde nach Zusatz von Wasser mehrfach mit Pentan extrahiert. Die methanolisch-wäßrige Fraktion wurde mit Trifluoracetanhydrid derivatisiert. Das GC-Massenspektrum der Pentanfraktion wies zwei Hauptpeaks auf. Der Peak bei m/z 184 wurde als 2-Decensäuremethylester **2** identifiziert, während der zweite Peak bei m/z 297 aufgrund charakteristischer Fragmentierungen dem N-(2-Decenoyl)-leucin-methylester **3** zugeordnet wurde. Mit der Identifizierung von **3** im Methanolysat von **1** war die Verknüpfung von C-12 und C-5 über N-1 im nativen Molekül aufgeklärt. Aufgrund der ¹³C-chemischen Verschiebungen ergab sich nur eine Möglichkeit für die Anordnung des verbliebenen Fragmentes sowie der Carbonylgruppe C-2. Die Formulierung der Struktur von **1** als 3-Acetyltetramsäure wurde unter anderem bestätigt durch die Identifizierung von Verbindung **4** als Hauptkomponente der methanolisch-wäßrigen Fraktion des Methanolysates. Die Deacylierung von 3-Acyltetramsäuren unter sauren Bedingungen ist seit langem bekannt, ebenso ist die O-Alkylierung von Tetramsäuren belegt. Die absolute Konfiguration des ebenfalls in der methanolischen Fraktion detektierten N-Trifluoracetylleucinmethylesters **5** (bestimmt durch GC-MS-Analyse an Chirasil-Val) läßt auf die (5R)-Konfiguration von **1** schließen. Die Methanolyseprodukte **3** und **5**, deren Identifizierung entscheidend zur Strukturaufklärung von **1** beitrug, sind insofern außergewöhnlich, als daß ihre Bildung die Spaltung einer C-C-Bindung erfordert. Im Zuge der Strukturaufklärung bislang isolierter 3-Acyltetramsäuren wurde die Freisetzung einer Aminosäure stets durch Oxidationsmittel erreicht.

Die Formulierung von **1** als 3-Acetyl-1-(2-Decenoyl)-(5R)-isobutyl-delta2-pyrrolin-4-on entspricht dem nach Analyse der ¹³C-chemischen Verschiebungen (Tabelle 1) in Acetonitril überwiegend (60 %) vorliegenden Tautomer **1a.** Die ¹³C-NMR-Daten des zweiten Signalsatzes sprechen für ein ausgeglichenes Verhältnis der internen Tautomere **1b** (20 %) und **1c** (20 %). Damit unterscheidet sich **1** bezüglich seiner Tautomerieverhältnisse von allen anderen natürlich auftretenden 3-Acyltetramsäuren, welche in Lösung nahezu ausschließlich die Pyrrolidin-2,4-dion-Form bevorzugen. Die exzeptionelle Präferenz der delta₂-Pyrrolin-4-on-Form in **1** ist auf den 2-Decenoylsubstituenten an N-1 zurückzuführen, welcher nicht nur die H-Brückenakzeptoreigenschaften der Carbonylgruppe C-2 reduziert, sondern auch die endozyklische Doppelbindung durch Konjugation stabilisiert. Im Hinblick auf die in der Natur auftretenden Tetramsäuren, die sich durch ein breites Spektrum biologischer Aktivität auszeichnen, ist die Struktur von **1** in chemischer Hinsicht interessant durch die einzigartige Acylierung des Ringstickstoffes mit einer alpha,beta-ungesättigten Fettsäure.

Bei der Strukturaufklärung wurde auch die Chiralität an Position 5 durch Totalhydrolyse untersucht. Als Hydrolyseprodukt wurde fast ausschließlich D-Leucin erhalten, was den Schluß zuläßt, daß Reutericyclin enantiomerenrein als 5R-1-(2-trans-Decenoyl)-3-acetyl-5-isobutyl-delta2-pyrrolin4-on vorliegen muß.

**Tabelle 1:**

| ¹H- und ¹³C-chemische Verschiebungen von Reutericyclin sowie beobachtete HMBC-Korrelationen ([D₃]Acetonitril, 298 K). Aufgrund der auftretenden Keto-Enol-Tautomerie wurden für einige Kerne zwei Signalsätze detektiert: **1a** zu 60 %, **1b** und **1c** zu jeweils 20 %. | | | | | |
|---|---|---|---|---|---|
| | δ(¹³C) | | δ(¹H) | | HMBC-Korrelationen |
| | **1a** | **1b/1c** | **1a** | **1b/1c** | |
| 2 | 167.3 | 174.8 | - | | - |
| 3 | 106.1 | 103.6 | - | | 11-H |
| 4 | 199.2 | 194.5 | - | | 5-H, 6-H |
| 5 | 60.2 | 64.1 | 4.68 | 4.41 | 6-H |
| 6 | 39.8 | | 1.81 | 1.76 | 5-H, 7-H, 8-H, 9-H |
| 7 | 25.2 | | 1.82 | | 5-H, 6-H, 8-H, 9-H |
| 8 | 23.8 | | 0.88 | | 9-H |
| 9 | 22.6 | | 0.93 | | 8-H |
| 10 | 195.6 | 189.3 | - | | 11-H |
| 11 | 22.8 | 20.2 | 2.51 | | - |
| 12 | 165.9 | 165.5 | - | | 13-H, 14-H |
| 13 | 124.2 | | 7.29 | 7.22 | 15-H |
| 14 | 150.7 | 151.8 | 7.06 | 7.10 | 15-H, 16-H |
| 15 | 33.3 | 33.2 | 2.28 | 2.29 | 13-H, 14-H, 16-H |
| 16 | 28.8 | | 1.50 | | 14-H, 15-H |
| 17 | 29.8 | | 1.34 | | 15-H, 16-H, 18-H |
| 18 | 29.8 | | 1.34 | | 16-H, 17-H |
| 19 | 32.5 | | 1.30 | | 20-H, 21-H |
| 20 | 23.3 | | 1.32 | | 19-H, 21-H |
| 21 | 14.3 | | 0.90 | | 20-H |

### Beispiel 3: Synthese von Reutericyclin

Um ausreichende Mengen des Naturstoffs und seiner Analoga zu gewinnen, wurde eine Synthese dieser neuartig substituierten Tetramsäure entwickelt. Außerdem sollte die Übereinstimmung der NMR-spektroskopischen Daten des synthetischen 1-(2-trans-Decenoyl)-3-acetyl-5-isobutyl-delta₂-pyrrolin-4-ons mit denen des aus Lactobacillus isolierten Reutericyclins die Richtigkeit der Strukturaufklärung bestätigen.

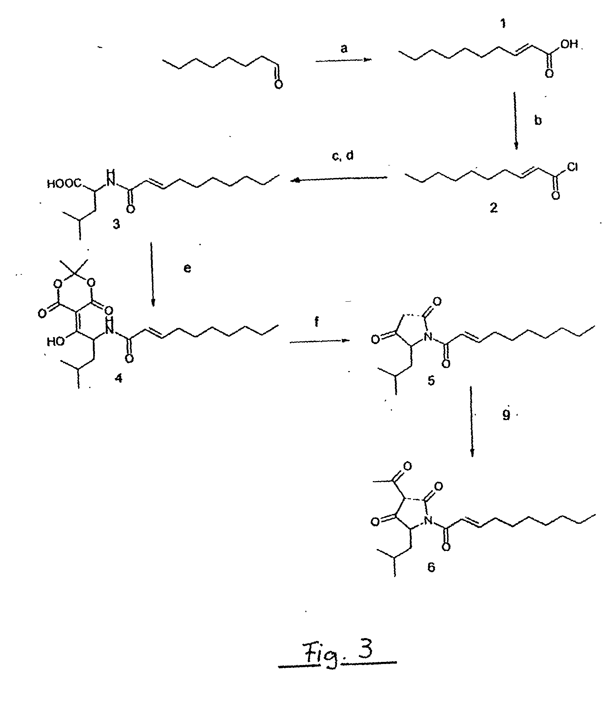

Reutericyclin wurde in 7-stufiger Synthese über eine C-Acylierung von Meldrumsäure mit (2-trans-Decenoyl)-Leucin, thermische Cyclisierung und anschließende C3-Acetylierung hergestellt (Figur 3).

Die alpha,beta-ungesättigte Carbonsäure **1** wird durch Knoevenagel-Kondensation des entsprechenden Aldehyds mit Malonsäure hergestellt und mit Thionylchlorid in das Carbonsäurechlorid **2** übergeführt. Die Acylierung des Leucin-tert-butylesters und anschließende Esterspaltung führt in sehr guter Ausbeute zur N-acylierten Aminosäure **3**. Die N-acylierte Tetramsäure **5** ist durch Kupplung von **3** mit Meldrumsäure (cycl.-Isopropylidenmalonat) und anschließende thermische Cyclisierung zugänglich. Die direkte Acylierung von **5** in Position 3 führt unter Verwendung von Carbonsäurechloriden in Gegenwart von Lewis-Säuren zu **6.**

Der Nachweis von **5** im Rohprodukt erfolgte durch HPLC-MS. Dadurch konnten alle Nebenprodukte und Verunreinigungen aufgeklärt werden. Von diesen waren keine störenden Einflüße auf die Folgereaktionen zu erwarten, deshalb wurde auf eine Aufreinigung von **5** vor der Acetylierung zu **6** verzichtet.

(Bei den Verunreinigungen bzw. Nebenprodukten handelt es sich um Dicyclohexylharnstoff, der offensichtlich nicht quantitativ ausfällt und somit nicht durch Filtration abgetrennt werden kann, um unumgesetztes N-Decenoylleucin **3** sowie dessen tBu-Ester. Die Bildung des tBu-Esters ist die Folge einer geringfügigen Verunreinigung von **3** mit tert-Butylalkohol, der bei der Esterspaltung anfällt und durch einmalige Gefriertrocknung nicht vollständig entfernt werden kann.)

Nach präparativer HPLC-Reinigung des Rohprodukts wurde **6** wie folgt mit Reutericyclin verglichen. Mit ES-FT-ICR-MS wurde eine hochgenaue Massenbestimmung durchgeführt:

Für das Syntheseprodukt **6** wurde 350,2326830 Da und für den Naturstoff 350,2325849 Da gemessen. Unter Berücksichtigung einer Abweichung von 0,002 m/z vom gemessenen Wert ist keine andere sinnvolle elementare Zusammensetzung für 6 (M+H⁺) möglich, als C₂₀H₃₂NO₄. Somit ist bewiesen, daß **6** und Reutericyclin dieselbe Summenformel besitzen.

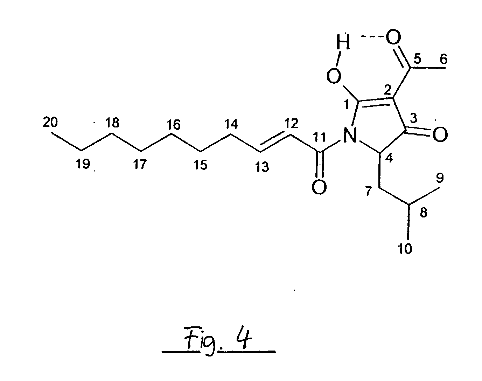

Die NMR-spektroskopischen Untersuchungen von **6** (Figur 4) ergaben ebenfalls eine völlige Übereinstimmung mit den Spektren des Naturstoffs Reutericyclin.

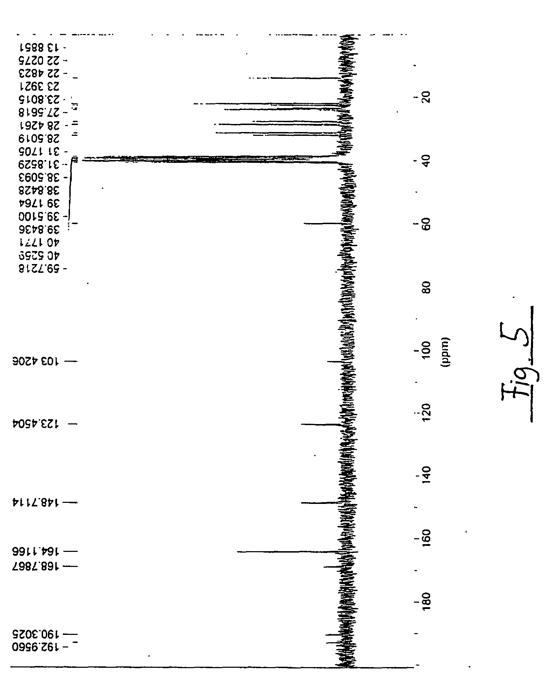

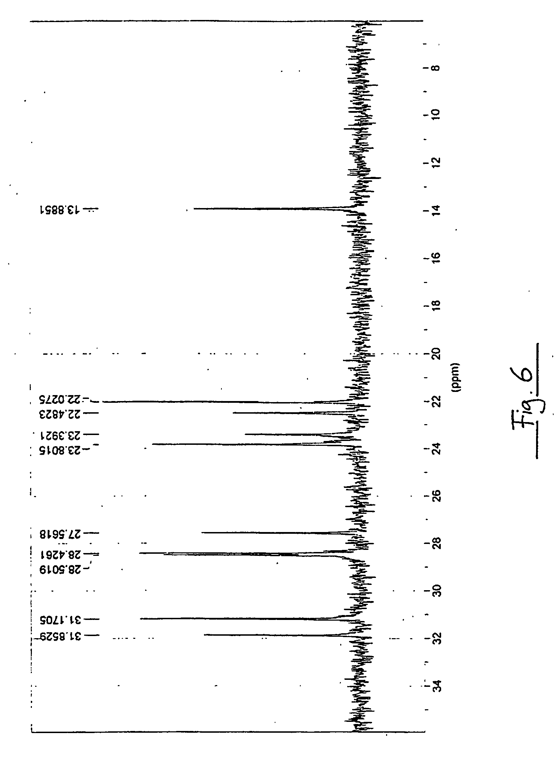

Das 1D-¹³C-NMR-Spektrum (Figur 5 und Figur 6) dient in erster Linie dem Nachweis der C-Signale, die nicht durch das HSQC-Experiment nachweisbar sind. Es handelt sich hierbei um die Signale von C-11, C-5, C-3 und C-1. Diese Signale wurden nachgewiesen.

Die exakten ¹H-Daten sowie die ¹³C-Signale, die nicht durch das 1D-Spektrum gewonnen werden konnten (C-6 und C-7), wurden durch das HSQC-Experiment ermittelt.

**Tabelle 2:**

| Vergleich der NMR-Daten des Naturstoffs Reutericyclin mit denen des Syntheseproduktes **6.** | | | | |
|---|---|---|---|---|
| | **Reutericyclin in DMSO-d**_{**6**} **isoliert aus LAB** | | **synthetisches 1-(2-*trans*-Decenoyl)-3- acetyl-5-isobutyl-Δ**_{**2**}**-pyrrolin-4-on in DMSO-d**_{**6**} | |
| | **δ(**^{**1**}**H)** | **δ(**^{**13**}**C)** | **δ(**^{**1**}**H)** | **δ(**^{**13**}**C)** |
| 1 | - | 168,9 | - | 168,8 |
| 2 | - | 103,2 | - | 103,5 |
| **3** | - | 192,9 | - | 193,0 |
| **4** | 4,38 | 59,7 | 4,41 | 59,8 |
| **5** | - | 190,3 | - | 190,3 |
| **6** | 2,39 | 24,0 | 2,41 | 23,9 |
| **7** | 1,69 | 38,6 | 1,68 | 38,3 |
| **8** | 1,70 | 23,7 | 1,68 | 23,8 |
| **9** | 0,83 | 22,5 | 0,83 | 22,5 |
| **10** | 0,79 | 23,3 | 0,79 | 23,4 |
| **11** | - | 164,1 | - | 164,1 |
| **12** | 7,27 | 123,5 | 7,26 | 123,5 |
| **13** | 6,92 | 148,4 | 6,93 | 148,7 |
| **14** | 2,21 | 31,8 | 2,21 | 31,9 |
| **15** | 1,42 | 27,5 | 1,41 | 27,6 |
| **16** | 1,27 | 28,4 | 1,27 | 28,5 |
| **17** | 1,27 | 28,3 | 1,25 | 28,4 |
| **18** | 1,25 | 31,1 | 1,24 | 31,2 |
| **19** | 1,23 | 22,0 | 1,23 | 22,1 |
| **20** | 0,84 | 13,8 | 0,84 | 13,9 |

Die NMR-Daten des Syntheseproduktes 6 stimmen mit denen des natürlichen Reutericylins vollständig überein.

### Experimenteller Teil:

### 1.1 trans-2-Decensäure 1

Oktanal (3 mmol) wird mit Malonsäure (3 mmol) in Pyridin (3 ml) auf 75 °C erhitzt, bis kein Kohlendioxid mehr gebildet wird (ca. 48 h). Die abgekühlte Mischung wird mit 10 % iger Salzsäure angesäuert und mit Diethylether (5 x 5 ml) extrahiert. Der vereinigte Extrakt wird eingeengt. Der ölige, schwach gelbliche Rückstand wird in Diethylether (5 ml) aufgenommen und mit 10-proz. Natronlauge (5 x 3 ml) extrahiert. Der Extrakt wird mit 10-proz. Salzsäure angesäuert und mit Diethylether (5 x 5 ml) extrahiert. Der Extrakt wird getrocknet über Magnesiumsulfat, filtriert und eingeengt. Die reine trans-2-Decensäure fällt an als schwach gelbliches Öl.
EI-MS: 171 m/z (M+H⁺); Ausbeute: 60 % d. Th..

### 1.2 trans-2-Decensäurechlorid 2

trans-2-Decensäure wird mit Thionylchlorid umgesetzt. Das überschüssige Thionylchlorid wird im Vakuum entfernt. Das Säurechlorid fällt als dunkelbraunes Öl an und wird ohne weitere Aufreinigung umgesetzt.
FD-MS: 153 m/z (M-Cl⁻), 188 m/z bzw. 190 m/z (M⁺); Ausbeute: 100 % d. Th..

### 1.3 Darstellung von N-trans-2-Decenoylleucin 3

Leucin-tert.-butylester-Hydrochlorid (3 mmol) und Diisopropylethylamin (6 mmol) werden in trockenem Dichlormethan (5 ml) unter Feuchtigkeitsausschluß tropfenweise mit trans-2-Decensäurechlorid (3 mmol) versetzt. Nach 3 h Rühren bei 40 °C werden Trifluoressigsäure (6 ml) und Wasser (500 µl) zugegeben. Nach weiteren 3 h bei 40 °C wird die abgekühlte Mischung mit 10-proz. Salzsäure (5 x 10 ml) gewaschen. Die organische Phase wird eingeengt. Der ölige, dunkelbraune Rückstand wird in einer Acetonitril/Wasser-Mischung aufgenommen und gefriergetrocknet.
EI-MS: 284 m/z (M+H⁺); Ausbeute: 90 % d. Th..

### 1.4 Darstellung von N-trans-2-Decenoyl-3-acetyl-5-iso-butylpyrrolidin-2,4-dion 6

N-trans-2-Decenoylleucin (3 mmol), cycl.-Isopropylidenmalonat (3 mmol), 4-Dimethylaminopyridin (5 mmol) werden in Dichlormethan (5 ml) unter Feuchtigkeitsausschuß und Rühren tropfenweise mit Dicyclohexylcarbodiimid (3,5 mmol) in Dichlormethan (2 ml) versetzt. Nach 5 h wird der Dicyclohexylharnstoff abfiltriert und das Filtrat wird in Ethylacetat (25 ml) aufgenommen. Die organische Phase wird mit 5-proz. Kaliumhydrogensulfatlösung (3 x 8 ml), Wasser (3 x 8 ml) und gesättigter, wässriger Natriumchloridlösung (3 x 8 ml) gewaschen und eingeengt. Der Rückstand wird mit Methanol (3 ml) 1 h unter Rückfluß erhitzt. Nach Einengen resultiert ein hochviskoser, rotbrauner Rückstand, der im Vakuum 24 h getrocknet wird.

Der hochviskose Rückstand wird in Nitrobenzol (3 ml) aufgenommen und unter Fcuchtigkeitsausschluß werden unter Rühren Acetylchlorid (5 mmol) und 1 molare Titantetrachloridlösung (6 ml) in Dichlormethan zugegeben. Nach 3 h Rühren bei 50 °C wird die Mischung in salzsaures Eiswasser (30 ml) gegeben. Die organische Phase wird abgetrennt und die wässrige Phase wird mit Dichlormethan (3 x 10 ml) extrahiert. Die organische Phase und die Extrakte werden vereinigt, gewaschen mit gesättigter Natriumhydrogencarbonatlösung (3 x 10 ml), gefiltert und unter reduziertem Druck bei 50 °C eingeengt. Die extrem lipophile Verbindung **6** läßt sich durch präparative HPLC aus dem öligen, dunkelbraunen Rückstand isolieren. HPLC-, NMR-, und MS-Daten sind übereinstimmend mit natürlichem Reutericyclin (Tab. 2).
Ausbeute: 15 %.

### Beispiel 4: Totalhydrolyse von Reutericyclin

Die Totalhydrolyse des Syntheseproduktes Reutericyclin **6** lieferte als Hydrolyseprodukte zu annähernd gleichen Anteilen D-und L-Leucin. Da die Synthese von **6** vom L-Enantiomer des Leucin-tert-butylesters ausging, ist dieses Ergebnis gleichbedeutend mit einer vollständigen Razemisierung, die wahrscheinlich während der Umsetzung von **3** nach **4** (Beispiel 3) stattgefunden hat. N-acylierte Aminosäuren razemisieren bekanntlich leichter als urethangeschützte Derivate.

### Beispiel 5: Antibakterielle Wirkung von Reutericyclin

Das Wirkungsspektrum von Reutericyclin wurde mit Hilfe des Naturstoffes untersucht, der aus Lactobacillus reuteri LTH 2584 wie oben beschrieben isoliert wurde. Die antimikrobielle Aktivität wurde gegenüber einer Vielzahl unterschiedlicher Bakterien getestet. Hierfür wurden Verdünnungstest auf Mikrotiterplatten durchgeführt. Kulturen der verschiedenen Bakterien wurden mit Verdünnungen der erfindungsgemässen Verbindung behandelt und die Konzentration bestimmt, die eine Hemmung des Wachstums der Bakterien im Vergleich mit Kontrollen beobachten ließ.

Diese sogenannte minimale inhibitorische Konzentration wurde für das Bakterium Lactobacillus sanfranciscensis (ATCC27651) mit 0,1 +/-0,05 mg/l bestimmt. Andere Laktobacillusarten, wie beispielsweise L. curvatus (LTH1174) oder L. sakei (LTH673) zeigten sehr ähnliche Empfindlichkeiten gegenüber Reutericyclin.

Einige humanpathogene Keime, wie beispielsweise Enterococcus Faecalis (DSM20409) erwies sich sogar noch als deutlich empfindlicher gegenüber Reutericyclin. Hier bewirkte eine Konzentration von etwa 0,05 mg/l eine Hemmung des Wachstums der Bakterien. Interessanterweise zeigten Stämme dieser Bakterienart, die als Antibiotika-resistente klinische Isolate vorlagen, ebenfalls eine Empfindlichkeit gegenüber Reutericyclin. Es war also keine Kreuzresistenz festzustellen.

Andere humanpathogene Keime, wie Listeria ivanovii (DSM20750) oder Staphylocokus aureus (SG511) zeigten Empfindlichkeiten gegenüber Reutericyclin in einer entsprechenden Größenordnung wie Lactobacillus sanfranciscensis.

Gram-negative Bakterien, beispielsweise Escherichia coli, waren ebenso wie Hefen nicht empfindlich gegenüber Reutericyclin und ließen sich durch die getesteten Konzentrationen nicht in ihrem Wachstum wesentlich beeinflußen.

Die antibakterielle Wirkung des Syntheseproduktes **6** war etwas schwächer als für das natürliche Reutericyclin festgestellt worden war. Dies liegt vermutlich daran, daß **6** im Gegensatz zum natürlichen Reutericyclin als Razemat vorliegt. In der Regel führten doppelte Mengen des Syntheseprodukts zu gleichen Ergebnissen wie eine Menge des "natürlichen" Reutericyclins.

## Patentansprüche

1. Verbindung der Formel I, 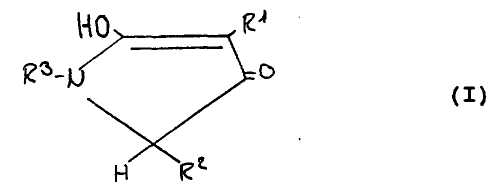 wobei
R¹ ein Acylrest, vorzugsweise der Acylrest einer organischen Carbonsäure ist,
R² ein Alkylrest, vorzugsweise ein C₁ bis C₅-Alkyl ist, und
R³ ein Acylrest einer organischen Carbonsäure ist.

2. Verbindung nach Anspruch 1, **dadurch gekennzeichnet, daß** sie als Racemat vorliegt.

3. Verbindung nach Anspruch 1, **dadurch gekennzeichnet, daß** sie im wesentlichen als ein Enantiomer vorliegt.

4. Verbindung nach einem der vorhergehenden Ansprüche, **dadurch gekennzeichnet, daß** R¹ ein Acetylrest ist.

5. Verbindung nach einem der vorhergehenden Ansprüche, **dadurch gekennzeichnet, daß** R² ein Butylrest, vorzugsweise ein Isobutylrest ist.

6. Verbindung nach einem der vorhergehenden Ansprüche, **dadurch gekennzeichnet, daß** R³ der Acylrest einer längerkettigen Carbonsäure, vorzugsweise einer C₈ bis C₁₂-Carbonsäure ist.

7. Verbindung nach einem der vorhergehenden Ansprüche, **dadurch gekennzeichnet, daß** R³ der Acylrest einer α,β-ungesättigten Carbonsäure ist.

8. Verbindung nach einem der vorhergehenden Ansprüche, **dadurch gekennzeichnet, daß** R³ Decenoyl, vorzugsweise 2-trans-Decenoyl ist.

9. Verbindung nach einem der vorhergehenden Ansprüche, **dadurch gekennzeichnet, daß** die Verbindung 1-(2-trans-Decenoyl)-3-acetyl-5-isobutyl-Δ₂-pyrrolin-4-on ist.

10. Verbindung nach einem der vorhergehenden Ansprüche, **dadurch gekennzeichnet, daß** die Verbindung 5R-1-(2-trans-Decenoyl)-3-acetyl-5-isobutyl-Δ₂-pyrrolin-4-on ist.

11. Verwendung einer Verbindung nach einem der Ansprüche 1 bis 10 als Antibiotikum.

12. Verwendung nach Anspruch 11, **dadurch gekennzeichnet, daß** die Verbindung gegen das Wachstum von Gram-positiven Bakterien eingesetzt wird.

13. Verwendung nach Anspruch 11 oder Anspruch 12, **dadurch gekennzeichnet, daß** die Verbindung gegen das Wachstum von Vancomycin-resistenten Keimen eingesetzt wird.

14. Verwendung einer Verbindung nach einem der Ansprüche 1 bis 10 in der Lebensmitteltechnologie, insbesondere zur Herstellung von probiotischen Lebensmitteln.

15. Verwendung nach Anspruch 14, **dadurch gekennzeichnet, daß** die Verbindung als Zusatz zu Lebensmitteln eingesetzt wird.

16. Verwendung nach einem der Ansprüche 11 bis 15, insbesondere nach Anspruch 11, **dadurch gekennzeichnet, daß** die Verbindung in Konzentrationen von 0,01 bis 1 mg/l, vorzugsweise 0,05 bis 0,15 mg/l, eingesetzt wird.

17. Verwendung einer Verbindung nach einem der Ansprüche 1 bis 10 zur Herstellung einer pharmazeutischen Zusammensetzung.

18. Pharmazeutische Zusammensetzung, **dadurch gekennzeichnet, daß** sie mindestens eine Verbindung nach einem der Ansprüche 1 bis 10 sowie vorzugsweise einen pharmazeutisch akzeptablen Träger, insbesondere einen Träger für die topische oder orale Verabreichung umfaßt.

19. Verwendung einer Verbindung nach einem der Ansprüche 1 bis 10 zur Herstellung eines probiotischen Lebensmittels.

20. Probiotisches Lebensmittel, **dadurch gekennzeichnet, daß** es mindestens eine Verbindung nach einem der Ansprüche 1 bis 10 umfaßt.

21. Verfahren zur Isolierung einer Verbindung nach einem der Ansprüche 1 bis 10, insbesondere von 5R-1-(2-trans-decenoyl)-3-acetyl-5-isobutyl-Δ₂-pyrrolin-4-on, **dadurch gekennzeichnet, daß** es aus Lactobacillus reuteri, vorzugsweise Lactobacillus reuteri LTH2584, gewonnen wird.

22. Verfahren zur Herstellung einer Verbindung nach einem der Ansprüche 1 bis 10, insbesondere der Verbindung nach Anspruch 9 oder 10, mindestens umfassend die folgende Schritte:
a) C-Acylierung von Meldrumsäure mit einer geeigneten N-acylierten Aminosäure, insbesondere (2-trans-Decenoyl)-Leucin,
b) thermische Cyclisierung und
c) C3-Acylierung, vorzugsweise C3-Acetylierung.

## Claims

1. Compound of the formula I, 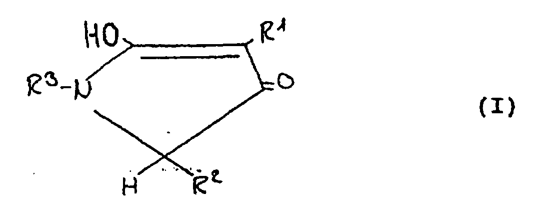 where
R¹ is an acyl radical, preferably the acyl radical of an organic carboxylic acid,
R² is an alkyl radical, preferably a C₁ to C₅ alkyl, and
R³ is an acyl radical of an organic carboxylic acid.

2. Compound according to Claim 1, **characterized in that** it is present as a racemate.

3. Compound according to Claim 1, **characterized in that** it is present essentially as one enantiomer.

4. Compound according to one of the preceding claims, **characterized in that** R¹ is an acetyl radical.

5. Compound according to one of the preceding claims, **characterized in that** R² is a butyl radical, preferably an isobutyl radical.

6. Compound according to one of the preceding claims, **characterized in that** R³ is the acyl radical of a relatively long-chain carboxylic acid, preferably a C₈ to C₁₂ carboxylic acid.

7. Compound according to one of the preceding claims, **characterized in that** R³ is the acyl radical of an α, β-unsaturated carboxylic acid.

8. Compound according to one of the preceding claims, **characterized in that** R³ is decenoyl, preferably 2-trans-decenoyl.

9. Compound according to one of the preceding claims, **characterized in that** the compound is 1-(2-transdecenoyl)-3-acetyl-5-isobutyl-Δ2-pyrrolin-4-one.

10. Compound according to one of the preceding claims, **characterized in that** the compound is 5R-1-(2-trans-decenoyl)-3-acetyl-5-isobutyl-Δ2-pyrrolin-4-one.

11. Use of a compound according to one of Claims 1 to 10 as antibiotic.

12. Use according to Claim 11, **characterized in that** the compound is used against the growth of Grampositive bacteria.

13. Use according to Claim 11 or Claim 12, **characterized in that** the compound is used against the growth of vancomycin-resistant microorganisms.

14. Use of a compound according to one of Claims 1 to 10 in food technology, in particular for producing probiotic foods.

15. Use according to Claim 14, **characterized in that** the compound is used as additive to foods.

16. Use according to one of Claims 11 to 15, in particular according to Claim 11, **characterized in that** the compound is used in concentrations of 0.01 to 1 mg/l, preferably 0.05 to 0.15 mg/l.

17. Use of a compound according to one of Claims 1 to 10 for producing a pharmaceutical composition.

18. Pharmaceutical composition **characterized in that** it comprises at least one compound according to one of Claims 1 to 10 and also, preferably, a pharmaceutically acceptable excipient, in particular an excipient for topical or oral administration.

19. Use of a compound according to one of Claims 1 to 10 for producing a probiotic food.

20. Probiotic food, **characterized in that** it comprises at least one compound according to one of Claims 1 to 10.

21. Process for isolating a compound according to one of Claims 1 to 10, in particular 5R-1-(2-transdecenoyl)-3-acetyl-5-isobutyl-Δ2-pyrrolin-4-one, **characterized in that** it is produced from Lactobacillus reuteri, preferably Lactobacillus reuteri LTH2584.

22. Process for producing a compound according to one of Claims 1 to 10, in particular the compound according to Claim 9 or 10, at least comprising the following steps:
a) C-acylation of Meldrum's acid with a suitable N-acylated amino acid, in particular (2-trans-decenoyl)leucine,
b) thermal cyclization and
c) C3-acylation, preferably C3-acetylation.

## Revendications

1. Composé de formule I, 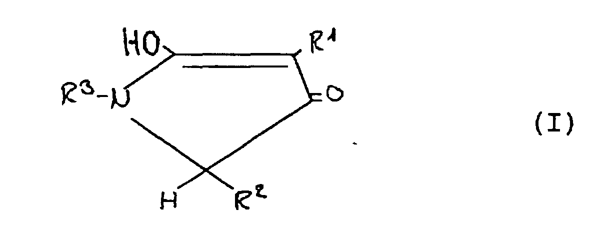 dans laquelle
R¹ est un radical acyle, de préférence le radical acyle d'un acide carboxylique organique,
R² est un radical alkyle, de préférence un alkyle en C₁ à C₅, et
R³ est un radical acyle d'un acide carboxylique organique.

2. Composé selon la revendication 1, **caractérisé en ce qu'**il se trouve sous forme de racémate.

3. Composé selon la revendication 1, **caractérisé en ce qu'**il se trouve essentiellement sous forme d'un enantiomère.

4. Composé selon l'une quelconque des revendications précédentes, **caractérisé en ce que** R¹ est un radical acétyle.

5. Composé selon l'une quelconque des revendications précédentes, **caractérisé en ce que** R² est un radical butyle, de préférence un radical isobutyle.

6. Composé selon l'une quelconque des revendications précédentes, **caractérisé en ce que** R³ est un radical acyle d'un acide carboxylique à chaîne longue, de préférence un acide carboxylique en C₈ à C₁₂.

7. Composé selon l'une quelconque des revendications précédentes, **caractérisé en ce que** R³ est le radical acyle d'un acide carboxylique α,β-insaturé.

8. Composé selon l'une quelconque des revendications précédentes, **caractérisé en ce que** R³ est un décénoyle, de préférence un 2-trans-décénoyle.

9. Composé selon l'une quelconque des revendications précédentes, **caractérisé en ce que** le composé est la 1-(2-trans-décénoyl)-3-acétyl-5-isobutyl-Δ₂-pyrrolin-4-one.

10. Composé selon l'une quelconque des revendications précédentes, **caractérisé en ce que** le composé est la 5R-1-(2-trans-décénoyl)-3-acétyl-5-isobutyl-Δ₂-pyrrolin-4-one.

11. Utilisation d'un composé selon l'une quelconque des revendications 1 à 10 en tant qu'antibiotique.

12. Utilisation selon la revendication 11, **caractérisée en ce que** le composé est utilisé contre la croissance de bactéries Gram-positives.

13. Utilisation selon la revendication 11 ou la revendication 12, **caractérisée en ce que** le composé est utilisé contre la croissance de germes résistants à la vancomycine.

14. Utilisation d'un composé selon l'une quelconque des revendications 1 à 10 dans la technologie alimentaire, en particulier pour la production de produits alimentaires probiotiques.

15. Utilisation selon la revendication 14, **caractérisée en ce que** le composé est utilisé en tant qu'additif pour des produits alimentaires.

16. Utilisation selon l'une quelconque des revendications 11 à 15, en particulier selon la revendication 11, **caractérisée en ce que** le composé est utilisé en concentrations de 0,01 à 1 mg/l, de préférence de 0,05 à 0,15 mg/l.

17. Utilisation d'un composé selon l'une quelconque des revendications 1 à 10 pour la production d'une composition pharmaceutique.

18. Composition pharmaceutique, **caractérisée en ce qu'**elle comprend au moins un composé selon l'une quelconque des revendications 1 à 10 ainsi que de préférence un support pharmaceutiquement acceptable, en particulier un support pour l'administration par voie topique ou orale.

19. Utilisation d'un composé selon l'une quelconque des revendications 1 à 10 pour la production d'un produit alimentaire probiotique.

20. Produit alimentaire probiotique, **caractérisé en ce qu'**il comprend au moins un composé selon l'une quelconque des revendications 1 à 10.

21. Procédé d'isolation d'un composé selon l'une quelconque des revendications 1 à 10, en particulier de la 5R-1-(2-trans-décénoyl)-3-acétyl-isobutyl-Δ₂-pyrrolin-4-one, **caractérisé en ce qu'**il est obtenu à partir de Lactobacillus reuteri, de préférence Lactobacillus reuteri LTH2584.

22. Procédé de préparation d'un composé selon l'une quelconque des revendications 1 à 10, en particulier du composé selon la revendication 9 ou 10, comprenant au moins les étapes suivantes :
a) C-acylation de l'acide de Meldrum avec un amino-acide N-acylé approprié, en particulier la (2-trans-décénoyl)leucine,
b) cyclisation thermique et
c) C3-acylation, de préférence C3-acétylation.
